# EUROPEAN PATENT APPLICATION

(11) **EP 4 708 328 A1**
(43) Date of publication of application: **11.03.2026**
(21) Application number: 24215438.3
(22) Date of filing: 26.11.2024
(51) Int. Cl.: G21C 1/22, G21C 17/06, G01K 13/12, G21C 17/112, G01N 25/04, G01N 33/00, G01N 1/12

(54) **SYSTEM FOR ESTIMATING CONCENTRATION OF NUCLEAR MATERIAL AND METHOD THEREFOR**

(30) Priority: 05.09.2024 KR 20240120887
(71) Applicant: X-Sentry Inc., Yeongdeungpo-gu, Seoul 07299 (KR)
(72) Inventor: Choi, Sungyeol, 08826 Seoul (KR); Yang, Wonseok, 08826 Seoul (KR); Park, Taehoon, 08826 Seoul (KR); Choi, Saehyun, 08826 Seoul (KR); Kim, Junwon, 08826 Seoul (KR); Kim, Jihun, 08826 Seoul (KR)
(74) Representative: Grünecker Patent- und Rechtsanwälte PartG mbB

(57) **Abstract**

The proposed invention relates to a system and method for estimating the concentration of a nuclear material present in a molten salt, wherein a sample of the molten salt is collected in a sampling cup (100), the sampling cup (100) is moved in a vertical direction by a driving means (200), a control part (300) controls the driving of the driving part (200), a temperature measuring apparatus (400) measures a temperature of a sampled molten salt and outputs temperature data, and a processing part (500) receives the temperature data showing a gradual cooling change for a predetermined time from the temperature measuring apparatus to estimate a concentration of a nuclear material present in the sampled molten salt.

## Description

### CROSS-REFERENCE TO RELATED APPLICATION(S)

This application claims priority from Korean Patent Application No. 10-2024-0120887, filed on September 5, 2024, in the Korean Intellectual Property Office, the disclosure of which is incorporated herein by reference in its entirety.

### BACKGROUND

### 1. Field

The following description relates to a technique for non-destructively estimating the concentration of a nuclear material in a molten salt.

### 2. Description of Related Art

Molten salts, which are used as nuclear fuel salts, are used in various energy industries due to their high heat capacity, high temperature stability, and high radioactivity stability. For example, molten salts are utilized in molten salt reactors, nuclear fusion reactor blankets, concentrated photovoltaic thermal energy storage devices, and the like. Among them, the nuclear fuel salt used as fuel for molten salt reactors is used by mixing alkali metal chlorides or fluorides and uranium chlorides or fluorides in a specific ratio.

In particular, under the circumstances of the recent growing need for carbon neutrality, molten salt reactors (MSRs) are being developed in various countries, and a real-time non-destructive analytical estimation technology for concentrations and base components dissolved in the molten salt of nuclear fuel is emerging as a core technology. Since the salt components in the MSR that uses liquid nuclear fuel change in real time, it is important to estimate and monitor nuclear materials (fissile materials) in the molten salt for economical and safe operation. In particular, for highly reliable quantitative management of nuclear materials, a quantitative concentration measurement technique for nuclear materials (for example, uranium) in a salt is essential.

However, non-destructive analytical techniques in the related art, such as electrochemical estimation techniques and spectroscopic estimation techniques, have limitations in estimating the concentration of high-concentration nuclear materials such as uranium and plutonium. For example, since uranium, as a main fissile material, occupies a high concentration of about 29 to 55 mol% in a molten salt fuel, it is difficult to apply electrochemical or spectroscopic measurement methods. Therefore, there is an urgent need for developing an inexpensive, accurate, and highly-reliable technique for estimating the concentration of a nuclear material.

The Korean Patent Publication (registration number: 10-1107095, "Real-time estimation device for uranium concentration in high-temperature molten salt") discloses a technique for real-time estimation of uranium concentration in a high-temperature molten salt capable of being directly used in a pyroprocess for recycling "spent nuclear fuel," which is capable of determining the concentration of trivalent and tetravalent uranium species using ultraviolet-visible light absorption spectroscopy, but does not disclose the content of estimating the concentration of nuclear material in nuclear fuel salt through the temperature change data of a cooling cup.

### SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

The following description relates to a non-destructive, inexpensive, accurate and highly-reliable technique for estimating the concentration of a nuclear material.

In one general aspect, a technique is disclosed for estimating the concentration of a nuclear material in components present in a molten salt in a molten salt reactor using the difference in temperature change that appears as the salt cools.

Other features and aspects will be apparent from the following detailed description, the drawings, and the claims.

### BRIEF DESCRIPTION OF THE DRAWINGS

FIG. 1 is a view illustrating a system for estimating the concentration of a nuclear material according to an embodiment.
FIG. 2 is a flow chart illustrating a method for estimating the concentration of a nuclear material according to an embodiment.
FIGS. 3 and 4 are views illustrating a method for estimating the concentration of a nuclear material according to an embodiment.
FIG. 5 is a set of graphs illustrating temperature change data generated from temperature data outputted by measuring the cooling temperature according to the concentration of UCl3 present in a molten salt in a sampling cup according to an embodiment.
FIG. 6 is a graph illustrating the temperature profile as a function of the concentration of UCl3 stored in advance.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

The following description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. Also, descriptions of well-known functions and constructions may be omitted for increased clarity and conciseness.

The above-described and additional aspects will be implemented through the embodiments described with reference to the accompanying drawings. It is to be understood that the elements of each embodiment can be variously combined in an embodiment or with elements of other embodiments, unless otherwise stated or mutually inconsistent. Terms used in the present specification and claims should be interpreted as meanings and concepts which are consistent with the described content or proposed technical spirit, based on the principle that an inventor can appropriately define the concept of the term to describe his/her own invention in the best manner. Hereinafter, preferred embodiments of the present invention will be described in detail with reference to the accompanying drawings.

### <Description of invention of claim 1>

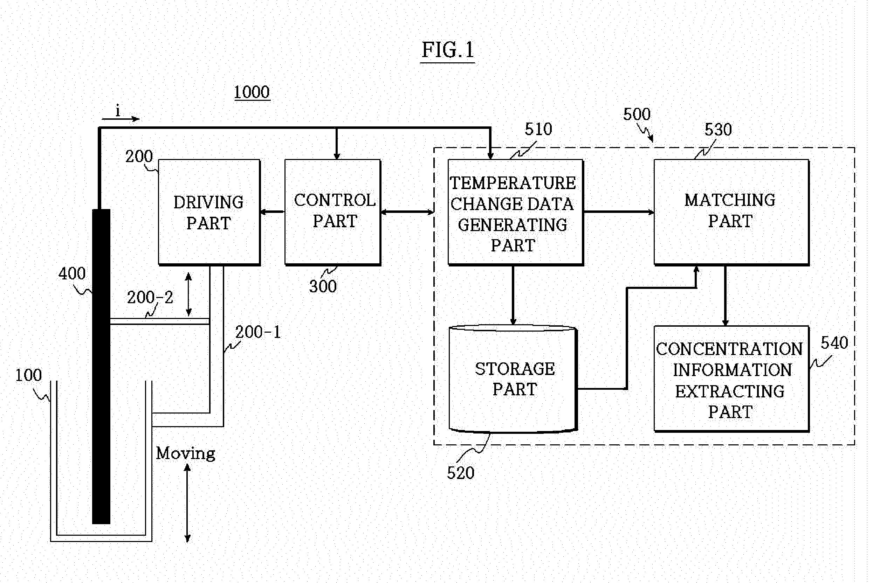

FIG. 1 is a view illustrating an automatic sampling device for a molten salt according to an embodiment. As illustrated, a system for estimating the concentration of a nuclear material 1000 includes a sampling cup 100, a driving part 200, a control part 300, a temperature measuring apparatus 400, and a processing part 500.

The sampling cup 100 may collect a sample of a molten salt. The sampling cup 100 may store a sample of a molten salt. The molten salt may refer to a liquid nuclear fuel contained in a nuclear reactor and may have a composition of, for example, NaCl-KCl-UCl3, NaCl-UCl3, KCl-UCl3, NaCl-MgCl2-UCl3, or NaF-KF-UF4, but is not limited thereto. The sampling cup 100 may have various shapes and may function as a container for temporarily storing the sampled molten salt for cooling.

The driving part 200 may drive the sampling cup 100 to move in the vertical direction. The driving part 200 may consist of an actuator such as a servo motor or a cylinder. The driving part 200 and the sampling cup 100 may be physically connected to a first connecting portion 200-1 to move the sampling cup 100 in the vertical direction by the driving of the driving part 200, and the sampling cup may also be moved in the front-back and left-right directions.

A cover gas is provided on top of the molten salt, and the sampling cup 100 may be moved to a cover gas region. The sampling cup 100 containing the molten salt in the cover gas region is cooled, so that the molten salt may be solidified. During the solidification process, the temperature measuring apparatus 400 may continuously record temperature information.

The control part 300 may control the driving of the driving part 200. The control part 300 may consist of a programmable logic controller (PLC), but is not limited thereto.

The temperature measuring apparatus 400 may continuously measure the temperature of the sampled molten salt and output temperature data. The temperature data may be converted into the form of current (i) and transmitted to the control part 300 and/or the processing part 500. The temperature measuring device 400 may be a thermocouple or a temperature sensor.

The processing part 500 may receive temperature data showing a gradual cooling change for a predetermined time from the temperature measuring apparatus to estimate the concentration of a nuclear material present in the sampled molten salt. The processing part 500 may consist of a computer. The nuclear material may be, for example, a compound including uranium, which is a fissile material, but is not limited thereto. Uranium, as a main fissile material, occupies a high concentration of about 29 to 55 ml% in the molten salt fuel, and limits electrochemical measurements, but the proposed invention may be suitable for concentration estimation.

### <Description of invention of claim 2>

According to an embodiment, the temperature measuring apparatus 400 is at least partially disposed inside the sampling cup 100 and may be moved in a vertical direction simultaneously with the sampling cup 100 by the driving of the driving part 200. The temperature measuring apparatus 400 can be attached to the inner sidewall of the sampling cup 100, but preferably may be disposed in the inner 'center region' of the sampling cup 100 to minimize temperature effects of the sampling cup 100 itself. For this purpose, the temperature measuring apparatus 400 and the driving part 200 may be physically connected to a first connecting portion 200-1 to move together as the sampling cup 100 moves in the vertical direction by the driving of the driving part 200.

However, the temperature measuring apparatus 400 may be moved independently of the movement of the sampling cup 100 and by another driving part (not illustrated). Further, the temperature measuring apparatus 400 may be stationary, and the sampling cup 100 may be moved to the stationary temperature measuring apparatus 400 by the driving of the driving part 200.

### <Description of invention of claim 3>

According to an embodiment, the control part 300 may control the processing part 500 to estimate the concentration of a nuclear material present in the sampled molten salt after the sampling cup 300 collects a sample of the molten salt. Therefore, the control part 300 may control the driving part 200 and the processing part 500, but the processing part 500 may also estimate the concentration of a nuclear material by directly receiving temperature data from the temperature measuring apparatus 400 without its processing operation being controlled by the control part 300.

### <Description of invention of claim 4>

According to an embodiment, the processing part 500 may include a temperature change data generating part 510, a storage part 520, a matching part 530, and a concentration information extracting part 540.

The temperature change data generating part 510 may receive temperature data showing a gradual cooling change for a predetermined time from the temperature measuring apparatus 400 to record the temperature change of the sampled molten salt and generate temperature change data. The temperature change data generating part 510 may receive the temperature data directly from the temperature measuring apparatus 400 or through the control part 300 .

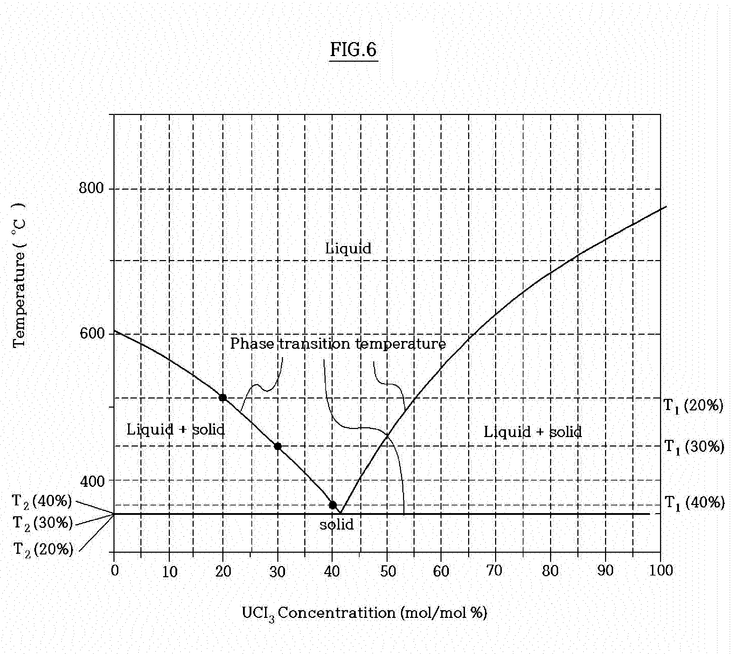

The storage part 520 may store in advance a temperature profile according to a change in concentration of a nuclear material present in the molten salt. FIG. 6 is a graph illustrating the temperature profile as a function of the concentration of UCl3 stored in advance. The storage part 520 may store such a temperature profile, as well as phase transition or phase change information.

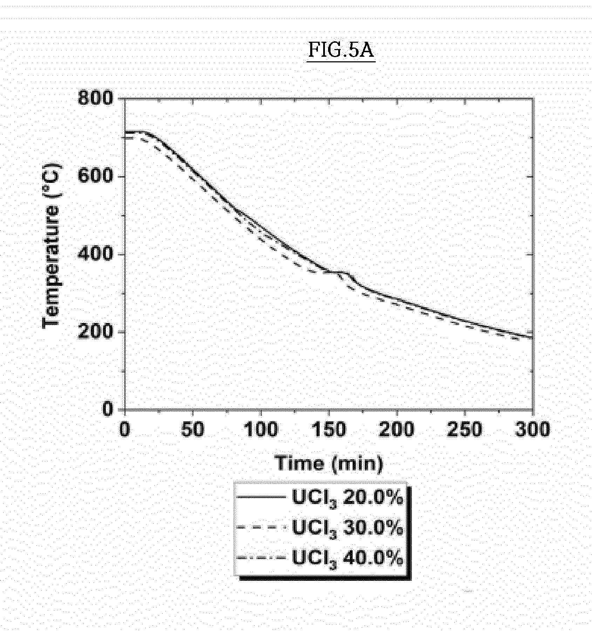

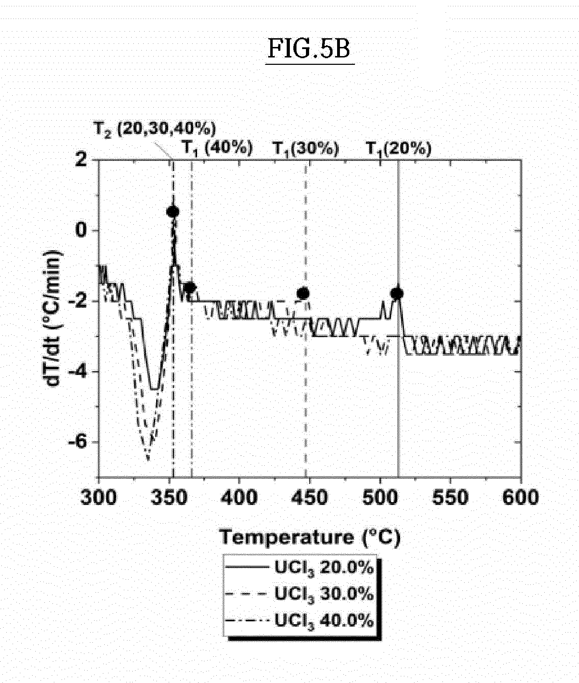

The temperature change data generated by the temperature change data generating part 510 may be the same as or similar to the graph illustrated in FIG. 5.

According to an embodiment, the storage part 520 may further store physical property information of the molten salt according to the state information of the molten salt. The physical property information or composition information of the molten salt may include at least one of the freezing point, boiling point, heat capacity, and latent heat of the molten salt. The storage part 520 may store information on the freezing point, boiling point, heat capacity, or latent heat of a molten salt having a particular composition at a particular temperature. The temperature change data generated by the temperature change data generating part 510 may also be stored in the storage part 520.

The matching part 530 may match the temperature change data to a temperature profile. Matching may mean comparing data to extract the corresponding data. The matching part 530 may match the temperature profile and the temperature change data stored in the storage part 520 to each other.

The concentration information extracting part 540 may extract nuclear material concentration information corresponding to the temperature change data from the temperature profile. For example, it is possible to extract information that the concentration of sampled UCl3 corresponds to 30 mol%.

### <Description of invention of claims 5 and 6>

According to an embodiment, the temperature change data generating part 510 may determine a phase transition temperature from the temperature change data.

According to an embodiment, the matching part 530 matches a phase transition temperature to a temperature profile, and the concentration information extracting part 540 extracts nuclear material concentration information corresponding to the phase transition temperature from the temperature profile.

The technique for determining the phase transition temperature from the temperature change data and matching the phase transition temperature to the temperature profile will be described in detail in the description of FIGS. 5 and 6.

### <Description of invention of claim 7>

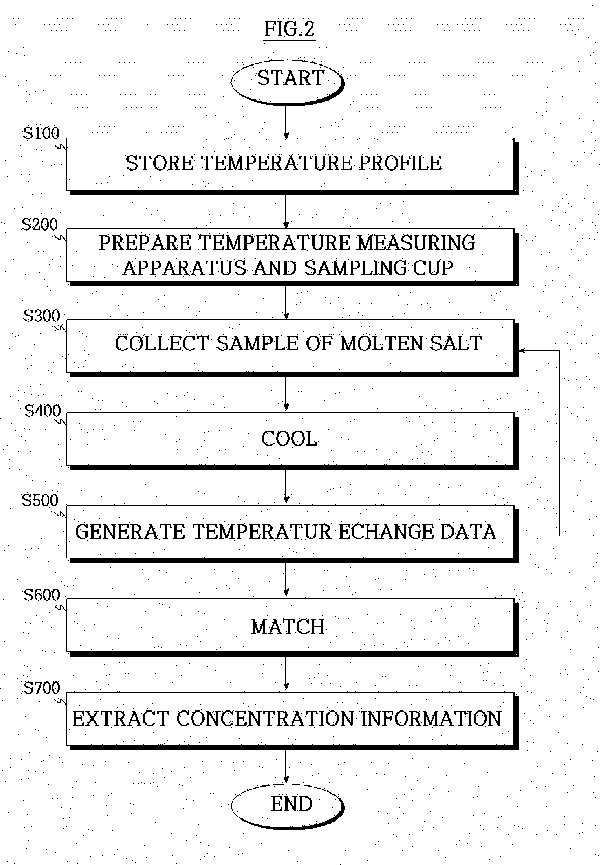

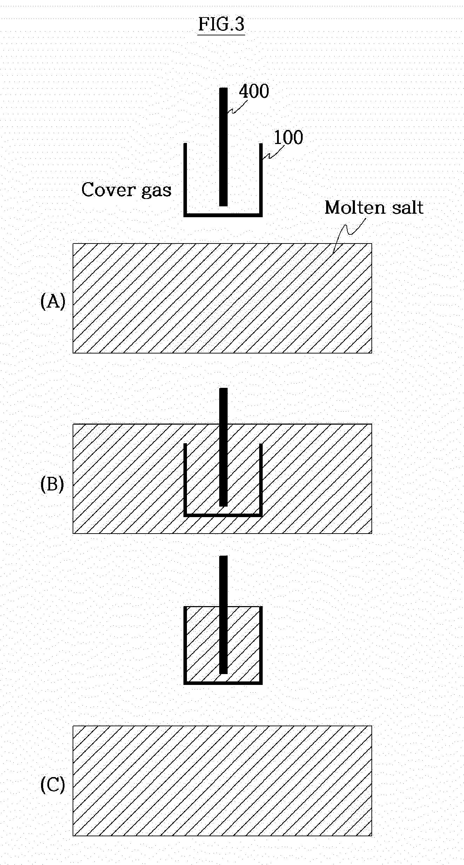

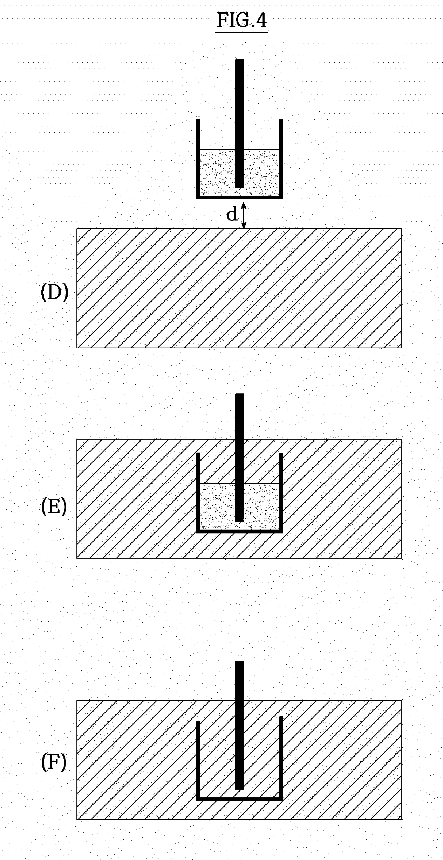

FIG. 2 is a flow chart illustrating a method for estimating a nuclear material concentration, according to an embodiment. FIGS. 3 and 4 are views illustrating a method for estimating a nuclear material concentration according to an embodiment.

As illustrated in FIG. 2, a nuclear material concentration estimation method S1000 for estimating the concentration of a nuclear material present in the molten salt provided at the bottom may include a temperature profile storage step S100, a temperature measuring apparatus and sampling cup preparation step S200, a molten salt sample collection step S300, a cooling step S400, a temperature change data generation step S500, a matching step S600, and a concentration information extraction step S700.

In the temperature profile storage step S100, a temperature profile according to a change in concentration of a nuclear material present in the molten salt may be stored in advance.

In the temperature measuring apparatus and sampling cup preparation step S200, a temperature measuring apparatus for measuring the temperature of the molten salt and outputting temperature data and a sampling cup for collecting the molten salt may be provided (see FIG. 3A). A cover gas may be present on top of the molten salt.

In the molten salt collection step S300, the sampling cup 100 may be moved in a vertical direction to collect a sample of the molten salt (see FIGS. 3B and 3C).

In the cooling step S400, the sampled molten salt may be gradually cooled for a predetermined time while being placed over the molten salt (see FIG. 4D). A constant distance (d) may be maintained between the sampling cup 100 and the molten salt. A cover gas is provided on top of the molten salt, and the sampling cup 100 may be moved to a cover gas region. The sampling cup 100 containing the molten salt in the cover gas region is cooled, so that the molten salt may be solidified (see FIG. 4D). During the solidification process, the temperature measuring apparatus 400 may continuously record temperature information.

In the temperature change data generation step S500, the temperature of the sampled molten salt that gradually changes as it is cooled for a predetermined time may be continuously measured by the temperature measuring apparatus, and temperature change data may be generated.

According to an embodiment, after the temperature change data generation step S500, a step of regenerating the temperature change data by returning to the molten salt sample collection step S300 may be repeated (see FIGS. 4E and 4F).

In the matching step S600, the temperature change data may be matched to the temperature profile. Matching may mean comparing data to extract the corresponding data.

In the concentration information extraction step S700, nuclear material concentration information corresponding to the temperature change data may be extracted from the temperature profile.

### <Description of invention of claims 8 and 9>

According to an embodiment, the temperature change data generation step S500 may further include a step of determining a phase transition temperature from the temperature change data.

According to an embodiment, the matching step S600 may further include a step of matching the phase transition temperature to the temperature profile, and the concentration information extraction step S700 may further include a step of extracting nuclear material concentration information corresponding to the phase transition temperature from the temperature profile.

FIG. 5 is a set of graphs illustrating temperature change data generated from temperature data outputted by measuring the cooling temperature according to the concentration of UCl3 present in a molten salt in a sampling cup according to an embodiment. FIG. 6 is a graph illustrating the temperature profile as a function of the concentration of UCl3 stored in advance. The present inventors carried out experiments using UCl3 as an example of the nuclear material. In FIG. 5, (A) is a graph showing temperature change data before the cooling temperature is differentiated with respect to time, and in FIG. 5, (B) is a graph showing the graph shown in (A) differentiated with respect to time to readily confirm the change in characteristics.

As illustrated in FIG. 5A, the difference in cooling rate due to the change in the concentration of UCl3 included in the molten salt in the sampling cup is shown. Specifically, it is illustrated that when the concentration of UCl3 is changed to 20%, 30%, and 40%, the (cooling) temperature characteristics also change. Theoretically, as the molten salt cools, it undergoes a phase transition from a liquid state to a state in which liquid and solid states coexist and to a solid state.

When the temperatures required to maintain each state are defined as the liquid temperature, phase transition temperature, and solidus temperature, theoretically the phase transition temperature has the characteristic that the rate of temperature change is relatively small (slow). The more diverse the components included in the molten salt, the more diverse the phase transition temperatures may be. The temperature change data may include phase transition temperature information.

As illustrated in FIG. 5B, a peak is shown at about 513°C when the concentration of UCl3 is 20% (UCl3 20.0%), a peak is shown at about 447°C when the concentration of UCl3 is 30% (UCl3 30.0%), and a peak is shown at about 366°C when the concentration of UCl3 is 40% (UCl3 40.0%). As described above, the temperature at a peak is the phase transition temperature. Once the phase transition temperature is determined, it may be matched to the graph shown in FIG. 6 to determine the currently measured concentration of UCl3. The graph shown in FIG. 6 shows a temperature profile, and specifically, the graph line shows the phase transition temperature characteristics according to the concentration of UCl3. As illustrated, it may be extracted that the concentration of UCl3 is 20% at about 513°C (T1 20%), 30% at about 447°C (30%), and 40% at about 366°C (40%). T2 indicates the phase transition temperature at which a substance transitions to a solid state regardless of concentration, and is calculated separately from T1 and may be used to estimate the concentration corresponding to T1. As described above, the concentration information of a specific nuclear material included in the molten salt may be extracted and estimated using the cooling temperature characteristics of the molten salt in the sampling cup.

According to the proposed invention, the concentration of a nuclear material can be estimated accurately and with highly reliability at low cost.

According to the proposed invention, the components of a nuclear material can be estimated accurately and with highly reliability at low cost, and remote and autonomous operation through nuclear fuel component analysis can be facilitated.

The effects of the present invention are not limited to the above-described effects, and other effects that have not been mentioned will be clearly understood by a person with ordinary skill in the art to which the present invention pertains from the present specification and the accompanying drawings.

Although the present invention has been described above through embodiments with reference to the accompanying drawings, it is to be interpreted that the present invention is not limited thereto and encompasses various modifications that can be obviously derived from these by those skilled in the art. The claims were intended to encompass these modifications.

Although all components constituting the embodiments of the present invention have been described as being combined to operate as one, the present invention is not necessarily limited to such embodiments. That is, within the scope of the present invention, one or more among all of the components may be selectively combined to operate as one.

## Claims

1. A nuclear material concentration estimation system for non-destructively estimating the concentration of a nuclear material present in a molten salt provided at a bottom, the system comprising:
a sampling cup for collecting a sample of a molten salt;
a driving part for driving such that the sampling cup is moved in a vertical direction;
a control part that controls the driving of the driving part;
a temperature measuring apparatus that continuously measures a temperature of a sampled molten salt and outputs temperature data; and
a processing part that receives the temperature data showing a gradual cooling change for a predetermined time from the temperature measuring apparatus to estimate a concentration of a nuclear material present in the sampled molten salt.

2. The nuclear material concentration estimation system of claim 1, wherein the temperature measuring apparatus is at least partially disposed inside the sampling cup and is moved in the vertical direction simultaneously with the sampling cup by the driving of the driving part.

3. The nuclear material concentration estimation system of claim 1, wherein the control part controls the processing part to estimate the concentration of a nuclear material present in the sampled molten salt after the sampling cup collects the sample of the molten salt.

4. The nuclear material concentration estimation system of claim 3, wherein the processing part comprises:
a temperature change data generating part that receives temperature data showing a gradual cooling change for a predetermined time from the temperature measuring apparatus to record the temperature change of the sampled molten salt and generate temperature change data;
a storage part in which a temperature profile corresponding to a change in concentration of a nuclear material present in molten salt is stored in advance;
a matching part that matches the temperature change data to the temperature profile; and
a concentration information extracting part that extracts nuclear material concentration information corresponding to the temperature change data from the temperature profile.

5. The nuclear material concentration estimation system of claim 4, wherein the temperature change data generating part determines:
a phase transition temperature from the temperature change data.

6. The nuclear material concentration estimation system of claim 5, wherein the matching part matches the phase transition temperature to the temperature profile, and
the concentration information extracting part extracts the nuclear material concentration information corresponding to the phase transition temperature from the temperature profile.

7. A nuclear material concentration estimation method for non-destructively estimating the concentration of a nuclear material present in a molten salt provided at a bottom, the method comprising:
a temperature profile storage step S100 of storing a temperature profile according to a change in concentration of a nuclear material present in the molten salt;
a temperature measuring apparatus and sampling cup preparation step S200 of preparing a temperature measuring apparatus for measuring a temperature of the molten salt and outputting temperature data, and a sampling cup for collecting a sample of the molten salt;
a molten salt collection step S300 of moving the sampling cup in a vertical direction to collect a sample of the molten salt;
a cooling step S400 of gradually cooling a sampled molten salt for a predetermined time while being placed over the molten salt;
a temperature change data generation step S500 of continuously measuring the temperature of the sampled molten salt which is gradually cooled and changed for a predetermined time by a temperature measuring apparatus and generating temperature change data;
a matching step S600 of matching the temperature change data to the temperature profile; and
a concentration information extraction step S700 of extracting nuclear material concentration information corresponding to the temperature change data from the temperature profile.

8. The nuclear material concentration estimation method of claim 7, wherein the temperature change data generation step S500 further comprises a step of determining a phase transition temperature from the temperature change data.

9. The nuclear material concentration estimation method of claim 8, wherein the matching step S600 further comprises a step of matching the phase transition temperature to the temperature profile; and
the concentration information extraction step S700 further comprises a step of extracting the nuclear material concentration information corresponding to the phase transition temperature from the temperature profile.
